# EUROPEAN PATENT SPECIFICATION

(11) **EP 3 088 027 B1**
(45) Date of publication and mention of the grant of the patent: **29.08.2018**
(21) Application number: 14874240.6
(22) Date of filing: 17.12.2014
(51) Int. Cl.: A61M 5/32, A61M 5/158, A61F 9/00

(54) **INJECTION NEEDLE**
INJEKTIONSNADEL
AIGUILLE D'INJECTION

(30) Priority: 24.12.2013 JP 2013265739
(43) Date of publication of application: 02.11.2016
(73) Proprietor: Yokohama City University, Yokohama-shi, Kanagawa 236-0027 (JP)
(72) Inventor: KADONOSONO, Kazuaki, Yokohama-shi Kanagawa 232-0024 (JP); FUTAMURA, Hideyuki, Tokyo 161-8525 (JP)
(74) Representative: Müller-Boré & Partner Patentanwälte PartG mbB
(86) International application number: PCT/JP2014/083383
(87) International publication number: WO 2015/098645

(56) References cited:
- WO-A1-2013/056588
- WO-A1-2014/036009
- JP-A- H03 503 373
- JP-A- 2001 502 211
- JP-A- 2002 291 883
- JP-A- 2002 291 884
- JP-A- 2008 154 842
- US-A1- 2010 256 597
- US-A1- 2012 191 064
- US-B1- 6 936 053
- US-B2- 7 217 263

## Description

### Technical Field

The present invention relates to an injection needle, and particularly to an injection needle suitable for a medical use.

### Description of Related Art

There is a retinal vein occlusion as one of eye diseases. The retinal vein occlusion is a disease that causes bleeding clogged veins of retina, due to a high blood pressure and arteriosclerosis, etc. This disease impairs the retina and causes symptoms such as a vison loss, etc. As a method of treating the retinal vein occlusion, the following method is known: a bleeding site or swelling of the retina is irradiated with laser beams so that the bleeding and the swelling are absorbed in the choroid side of a tissue under the retina, in addition to an oral medical treatment such as an anti-coagulant therapy. Further, the retinal vein occlusion includes a central vein occlusion and a branch vein occlusion. In these occlusions, arteriovenous intersections extravascular membrane sheath incision has been carried out for the branch vein occlusion. However, any one of the treatment methods is simply a symptomatic treatment.

Therefore, with an aim of a reproduction of a venous blood flow in the retina, there is proposed a treatment method of directly injecting a thrombolytic agent (t-PA) used for a treatment of cerebral infarction patients, into a vein (occluded vessel) of the retina. Such a treatment method is also called a causal therapy to eliminate the clogging of the blood vessel that is the cause of the retinal vein occlusion, and particularly in an aging society in which there are a large number of patients with the retinal vein occlusion, early establishment of the treatment method is strongly desired. Incidentally, as the injection needle for a medical use, for example the injection needle described in the following patent document 1 and patent document 2, is known.

### Prior Art Document

### Patent document

Patent document 1: Japanese Patent Laid Open Publication No.2006-280503
Patent document 2: Japanese Patent Laid Open Publication No.2004-290542

US 6 936 053 B1 discloses an apparatus for cannulating blood vessels, including a needle having a angled first end to define a sharp beveled tip and a second end that is connectable to a delivery device.

US 7 217 263 B2 discloses a microcathether system for infusion into retinal veins. The microcather system comprises flexible cannula arranged in a larger cannular, wherein the flexible cannula has a sharp distal end.

US 2010/025697 A1 discloses a microneedle device for infusion into the suprachoroidal space of the eye, comprising a hollow microneedle with an exit point in a tip portion. A device body is provided for holding themicroneedle.

US 2012/0191064 A1 discloses a cannula device for infusion of the choroid of a patient's eye.

### Summary of the Invention

### Problem to be solved by the Invention

The abovementioned method is the method of stabbing the retinal vein by the injection needle, and injecting a liquid for a treatment (referred to as "treatment liquid" hereafter) through the injection needle. Therefore, the injection needle capable of stabbing the retinal vein is required to be prepared. However, the thickness of the retinal vein (blood vessel) is ultrathin having a diameter of about 100 µm, and therefore the injection needle for stabbing the retinal vein must be formed further thin. Such an ultrathin injection needle can be made using a capillary tube made of glass,
but use of the capillary tube for the application of the injection needle for stabbing the thin vessel such as the retinal vein, involves the following problem.

Since the capillary tube is made of glass which is a brittle material, the capillary tube has a very fragile nature. Accordingly, if the capillary tube is broken or missing in a state of stabbing the blood vessel, there is a risk that a glass fragment remains in the blood vessel. Therefore, the capillary tube is not suitable for the abovementioned application.

Therefore, after strenuous efforts, there is provided the invention of realizing the injection needle capable of stabbing the ultrathin blood vessel by a needle tube, and injecting the treatment liquid into the blood vessel through the needle tube. Thus, the invention regarding the injection needle is provided (see Patent Application No.2012-230450)

Thereafter, regarding the injection needle of the prior application, further improvements are attempted by inventors of the present invention, wherein the inventors of the present invention initially consider that when the injection needle stabs the retinal vein, preferably a needle point stabs the blood vessel, with the injection needle tilted (inclined) along the blood vessel, similarly to a normal vascular injection. Therefore, the specification of the prior application discloses a constitution of bending a needle, as an embodiment. Further, regarding the shape, etc., of the needle point, similarly to the injection needle for a normal medical use, it is conceivable that a tilt angle of a blade surface with respect to a central axis of the injection needle is made small to about 20°, or the tilt angle of the blade surface is changed to two-step tilt.

However, it is found that as the injection needle used for the retinal vein occlusion, the injection needle having the abovementioned constitution is not necessarily preferable. Explanation will be given hereafter.

In the treatment of the retinal vein occlusion, the treatment liquid is injected into the retinal vein by stabbing the retinal vein by the injection needle at a prescribed angle (referred to as "stabbing angle" hereafter). At this time, when a tip side of the needle is set in a largely tilted state (in a state that the stabbing angle is small) by utilizing bending of the injection needle, the treatment liquid sometimes leaks to outside of the blood vessel without being efficiently injected into the blood vessel (vein). It is found that this is caused as follows: when the injection needle stabs the vein, a discharging port at a needle point portion for discharging the treatment liquid does not completely fit in the blood vessel, and due to protrusion of a part of the discharging port, the treatment liquid leaks to the outside of the blood vessel.

Such a trouble easily occurs when the tilt angle of the blade surface of the injection needle is small. The reason is as follows. First, when the tilt angle of the blade surface is small, a length of the blade surface becomes long in a central axis direction of the injection needle. When the length of the blade surface becomes long, the needle point easily interferes with a blood vessel wall at a deep side in a stabbing direction. Therefore, stabbing of the injection needle into the vein becomes shallow, and the discharging port is easily protruded from the blood vessel. Further, when a stabbing angle of the injection needle is set to be large with respect to the vein, the needle point further interferes with the blood vessel wall.

Under such a circumstance, after strenuous efforts by inventors of the present invention, the present invention is achieved regarding the constitution of the injection needle of the prior application, and particularly regarding the shape or a dimension, etc., of the needle tip portion.

A main object of the present invention is to provide the injection needle capable of stabbing the ultrathin blood vessel by a needle tube, and efficiently injecting the treatment liquid into the blood vessel through the needle tube.

### Means for solving the Problem

The invention is defined in the appended claims.

According to a first aspect there is provided an injection needle, including a needle tube having a discharging port at a needle point for discharging a treatment liquid,
the needle tube including a stabbing tube having a blade surface with the discharging port formed thereon, and a main needle tube which is thicker than the stabbing tube, with the stabbing tube provided on a tip portion of the main needle tube,
wherein the stabbing tube satisfies a dimension condition such that an outer diameter is 40 µm or more and 70 µm or less, and an inner diameter is 20 µm or more and 40 µm or less, and a tilt angle θ of the blade
surface with respect to a central axis of the stabbing tube satisfies a condition of 30° < θ ≤ 45°.

According to a second aspect there is provided the injection needle of the first aspect, wherein a length Lh of the blade surface in a central axis direction of the stabbing tube satisfies a condition of 30 µm ≤ Lh ≤ 120 µm.

According to a third aspect there is provided the injection needle of the first or second aspect, wherein a side face of the blade surface of the stabbing tube has a notched shape.

According to the invention, a position of the discharging port on the blade surface of the stabbing tube is biased to a needle point side.

According to a fifth aspect there is provided the injection needle of any one of the first to fourth aspects, wherein the needle tube is formed straight.

According to a sixth aspect there is provided an injection needle, including a needle tube having a discharging port at a needle point for discharging a treatment liquid,
the needle tube including a stabbing tube having a blade surface with the discharging port formed thereon, and a main needle tube which is
thicker than the stabbing tube, with the stabbing tube provided on a tip portion of the main needle tube,
wherein the stabbing tube satisfies a dimension condition such that an outer diameter is 70 µm or less (not including zero), and an inner diameter is 40 µm or less (not including zero), and a length Lh of the blade surface in a central axis direction of the stabbing tube satisfies a condition of 30 µm ≤ Lh ≤ 120 µm.

According to the present invention, the injection needle can be provided, which is capable of stabbing an ultrathin blood vessel, and efficiently injecting a treatment liquid into the blood vessel through the needle tube. Thus, it is possible to contribute to an early establishment of an effective treatment for a regeneration of a retinal venous blood flow of a retinal vein occlusion patient.

### Brief description of the drawings

FIG. 1 is a view showing a constitutional example of an injection needle according to an embodiment of the present invention.
FIG. 2 is a cross-sectional view of an essential part showing an attachment state of a stabbing tube.
FIG. 3 is a view showing a constitution of the stabbing tube.
FIG. 4 is a view showing a tilt angle of a blade surface.
FIG. 5 is a view showing a specific example of a case when the tilt angle of the blade surface is changed.
FIG. 6 is a view showing an example of a method of fabricating the stabbing tube.
FIG. 7 is a view showing a constitution of an essential part of the injection needle according to the present invention.
FIG. 8 is a view showing a modified example of the present invention.

### Detailed description of the Invention

Embodiments of the present invention will be described hereafter in detail, with reference to the drawings.

The embodiments of the present invention will be described in the following order.
1. Constitution of the injection needle
2. Method of manufacturing the injection needle
3. Effect of the embodiment
4. Other embodiment
5. Modified example, etc.

### <1. Constitution of the injection needle>

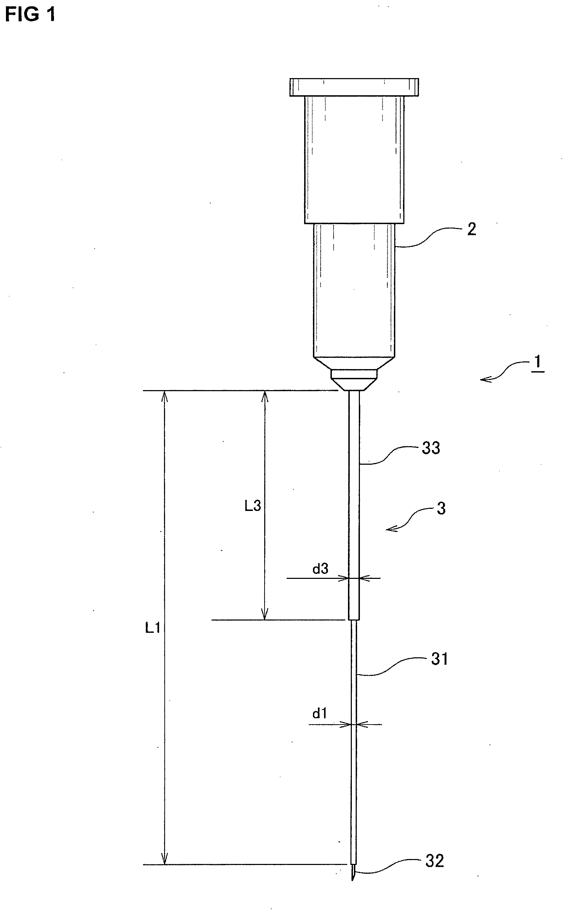

FIG. 1 is a view showing a constitutional example of the injection needle according to an embodiment of the present invention. An injection needle 1 shown in the figure, is mainly constituted of a needle base 2 and a needle tube 3. When the injection needle 1 is attached to a syringe not shown, the needle base 2 is a portion that is attachably/detachably fitted to a tip of the syringe. The needle base 2 is formed using thermoplastic resin such as polypropylene, polyethylene, or polyvinyl chloride. The needle base 2 is formed in a stepped cylindrical shape as a whole.

The needle tube 3 is attached to the tip portion of the needle base 2. The needle tube 3 is formed into thin tube shape, using a metal such as stainless steel, etc. The needle tube 3 is formed straight toward the needle point side from the needle base side. The needle tube 3 has a multistage constitution of a combination of a plurality of tubes with different dimensions (outer diameter, inner diameter, and a length) respectively. In this embodiment, as an example, the needle tube 3 has a three-stage constitution in which the main needle tube 31, the stabbing tube 32, and a reinforcement tube 33 are combined. When the needle tube 3 is constituted of the metal, it is possible to use a nickel chromium steel for example, other than the stainless steel.

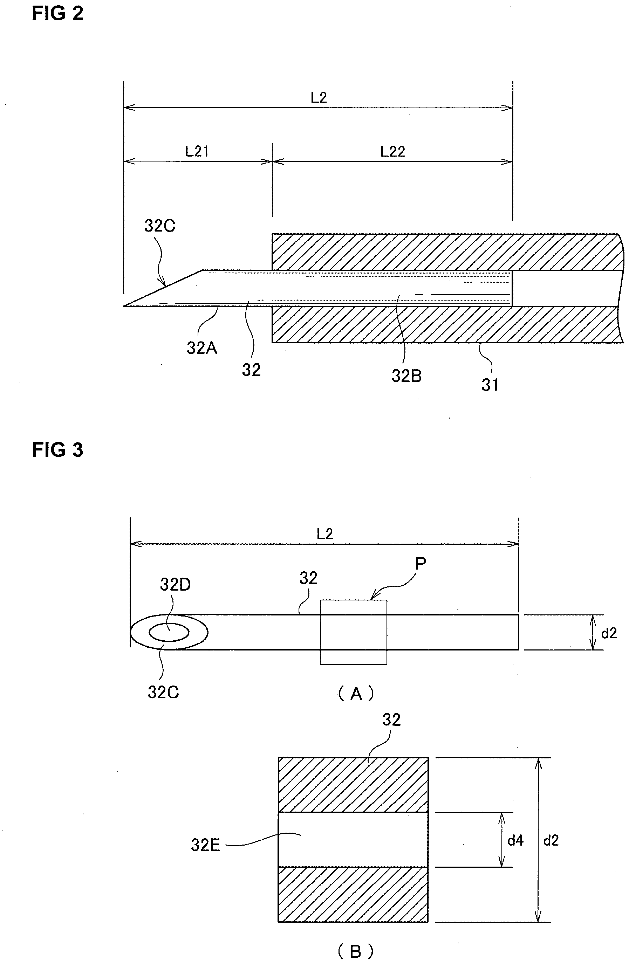

The main needle tube 31 is a longest tube among three tubes. Length L1 of the main needle tube 31 is defined as a protruding dimension from the tip portion of the needle base 2. The base part of the main needle tube 31 is fixed to the tip portion of the needle base 2 by adhesion, etc. Outer diameter d1 of the main needle tube 31 is set in a dimension larger than outer diameter d2 of the stabbing tube 32 (see FIG. 3), and smaller than outer diameter d3 of the reinforcement tube 33, and for example, set to 0.3 mm. The length L1 of the main needle base 31 is set to a dimension suitable for the application of the injection needle 1. This embodiment assumes the injection needle used for ophthalmic among various medical uses, and particularly for the purpose of stabbing into an eyeball to the retinal vein through the needle tube 3. Therefore, the length L1 of the main needle tube 31 is set, for example so as to secure a length of 25 mm or more (preferably around 27 mm) from the tip portion of the needle base 2, in consideration of a size of the eyeball.

FIG. 2 is a cross-sectional view of an essential part showing an attachment state of the stabbing tube, and FIG. 3 is a view showing the constitution of the stabbing tube. FIG. 3(B) is an expanded view showing a cross-sectional surface of part P of (A). The stabbing tube 32 is a shortest tube and a thinnest tube among the three tubes. The stabbing tube 32 is provided on the tip portion of the main needle tube 31. The base part of the stabbing tube 32 is concentrically fixed to the tip portion of the main needle tube 31. A blade surface 32C is provided on the tip portion (needle point) of the stabbing tube 32. The blade surface 32C is formed in a tilted state with respect to the central axis of the needle tube 3. The tilt angle of the blade surface 32C will be described in a later stage.

A part 32A of the stabbing tube 32 is protruded from the tip portion of the main needle tube 31, and the other portion 32B is inserted into the main needle tube 31. In the description hereafter, a part 32A of the stabbing tube 32 is referred to as a "protruding part 32A", and the other portion 32B is referred to as an "insertion part 32B". Length (total length) L2 of the stabbing tube 32 is set so as to satisfy a condition of preferably less than 7 mm (not including zero). The reason for applying this condition will be described later. Protruding dimension L21 of the protruding part 32A is set to 0.3 mm or more and 1.0 mm or less. The reason for setting the protruding dimension of the protruding part 32A to 0.3 mm or more, is as follows. When the protruding dimension L21 is shorter than 0.3 mm, the protruding part 32A is hidden in the shadow of a stepped portion due to a difference of the outer diameter between the main needle tube 31 and the stabbing tube 32, thus making it difficult to confirm the position of the needle point portion of the stabbing tube 32. However, when the tip portion of the main needle tube 31 is tapered, the protruding dimension L21 of the protruding part 32A can be set to 0.15 mm in consideration of the tilt of the tip of the stabbing tube 32. Further, the protruding dimension of the protruding part 32A is set to 1.0 mm or less, and this is because when the protruding dimension L21 is longer than 1.0 mm, the stabbing tube 32 succumbs to a resistance during stabbing (referred to as a "stabbing resistance" hereafter), and is likely to bend. An inserting dimension L22 of the insertion part 32B is preferably set to 0.5 mm or more and 3.0 mm or less, although depending on a processing method of the length of the stabbing tube 32 or the tip portion of the length L2, and the protruding dimension L21 of the protruding part 32A.

The abovementioned blade surface 32C is formed on the tip portion of the protruding part 32A. A discharging port 32D is formed on the blade surface 32C. The discharging port 32D is opened in-plane of the blade surface 32C. The discharging port 32D is the portion for discharging the treatment liquid by receiving a pressing force by a pressing means not shown. The treatment liquid is not particularly limited as long as it is the liquid used for a treatment, and a medicinal solution, physiological saline, and pure water, etc., can be given as examples. The discharging port 32D is formed at a most downstream part of a flow path 32E along the central axis of the stabbing tube 32 (see FIG. 3). The outer diameter d2 of the stabbing tube 32 is set to satisfy a condition of 70 µm or less (not including zero). The reason for applying this condition is as follows. When the outer diameter d2 of the stabbing tube 32 exceeds 70 µm, the stabbing tube 32 becomes excessively large compared with the blood vessel (retinal vein) having a target thickness of about 100 µm. In consideration of easiness of a procedure for stabbing the blood vessel by the stabbing tube 32, the outer diameter d2 of the stabbing tube 32 is preferably set to 60 µm or less. However, when the outer diameter d2 of the stabbing tube 32 is set to be excessively small, there is a problem that (1) the inner diameter d4 of the stabbing tube 32 is hardly secured, and (2) the stabbing tube 32 succumbs to the stabbing resistance and likely to bend, when the stabbing tube 32 is made of a metal. Therefore, the outer diameter d2 of the stabbing tube 32 is set to 40 µm or more and preferably set to about 50 µm.

The inner diameter (diameter of the flow path 32E) d4 of the stabbing tube 32 is set to satisfy the condition of 40 µm or less (not including zero). The reason for applying this condition is as follows. In a drawing process employed in the method of manufacturing the injection needle 1 (particularly the method of fabricating the stabbing tube 32) described later, the inner diameter d4 of the stabbing tube 32 is a dimension of about 60% of the outer diameter d2 of the stabbing tube 32, or slightly below 60%. The inner diameter d4 of the stabbing tube 32 is preferably large in consideration of simply flowability of the liquid, but it is preferably set to 20 µm or more and 30 µm or less in consideration of the outer diameter d2 of the stabbing tube 32. When the stabbing tube 32 is fabricated by the drawing process, a plurality of small irregularities (wrinkles by contraction due to the drawing process) are formed on an inner circumferential surface of the stabbing tube 32. In this case, the inner diameter d4 of the stabbing tube 32 is defined by an average diameter corresponding to an intermediary between a mountain portion and a valley portion of the abovementioned irregularities.

The reinforcement tube 33 is the thickest tube among the three tubes. The reinforcement tube 33 is attached to a root side of the needle tube 3 in the form of interpolating the main needle tube 31. The reinforcement tube 33 is provided for the purpose of reinforcing the needle tube 3, and particularly for increasing a rigidity of the needle tube 3 as a whole. The base part of the reinforcement tube 33 is fixed to the tip portion of the needle base 2 by adhesion, etc., together with the abovementioned main needle tube 31. The length L3 of the reinforcement tube 33 may be set to for example 1/5 or more and 2/3 or less, and more preferably may be set to 1/3 or more and 2/3 or less of the length L1 of the main needle tube 31. The length L3 of the reinforcement tube 33 is defined by the protruding dimension from the tip portion of the needle base 2.

The outer diameter d3 of the reinforcement tube 33 is set to for example 0.5 mm, although depending on the outer diameter d1 of the main needle tube 31. The inner diameter of the reinforcement tube 33 is set to be larger than at least the outer diameter d1 of the main needle tube 31, so that the main needle tube 31 can pass through the reinforcement tube 33. The reinforcement tube 33 is concentrically attached to outside of the main needle tube 31 so as to form a double tube constitution. The reinforcement tube 33 is not an essential element in the present invention, and is provided as needed, depending on the length or the thickness of the main needle tube 31.

### (Tilt angle of the blade surface)

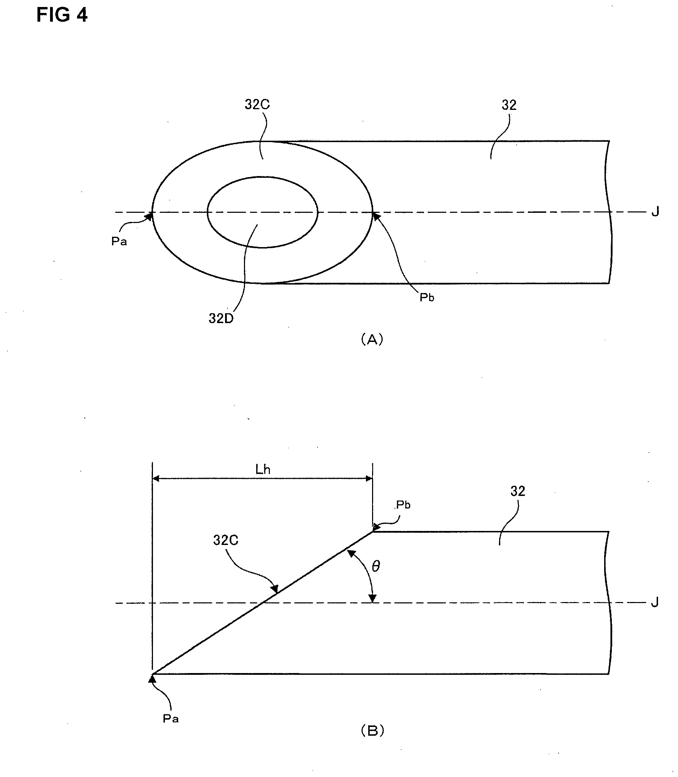

Here, the tilt angle of the blade surface 32C will be described, using FIG. 4(A) and FIG. 4(B).

As shown in the figure, a tilt angle θ of the blade surface 32C is defined by an angle formed by a central axis J of the stabbing tube 32 and the blase surface 32C. In the injection needle 1 of this embodiment, the tilt angle θ of the blasé surface 32C with respect to the central axis J of the stabbing tube 32 is set to satisfy a condition of 30° < θ ≤ 45°.

In the injection needle for a general medical use, the tilt angle of the blase surface is suppressed to about 20° at largest. The tilt angle of the blade surface is made small, mainly for making the stabbing resistance small. For the same reason, there is also an injection needle in which the tilt angle of the blade surface is changed to multiple stages (two stages in many cases). For example, in the injection needle in which the tilt angle of the blade surface is changed to two stages, the tilt angle at the needle point side is further made small so that a blade point portion becomes sharp.

On the other hand, in the injection needle of this embodiment, the tilt angle θ of the blade surface 32C of the stabbing tube 32 is larger than the injection needle for a general medical use by 10° or more. Further, the blade surface 32C of the stabbing tube 32 is formed not by a plurality of planar surfaces with different tilt angles but by one planar surface having the tilt angle in common. However, the blade surface 32C of the stabbing tube 32 may be the surface having an average tilt angle in common, and is not necessarily required to be formed by a single planar surface.

Further, as a constitution of the injection needle of this embodiment, in order to make the abovementioned stabbing resistance small, a side face of the blade surface 32C of the stabbing tube 32 may be formed in a notched shape (the shape of dropping the edge of the side face of the blade surface 32C).

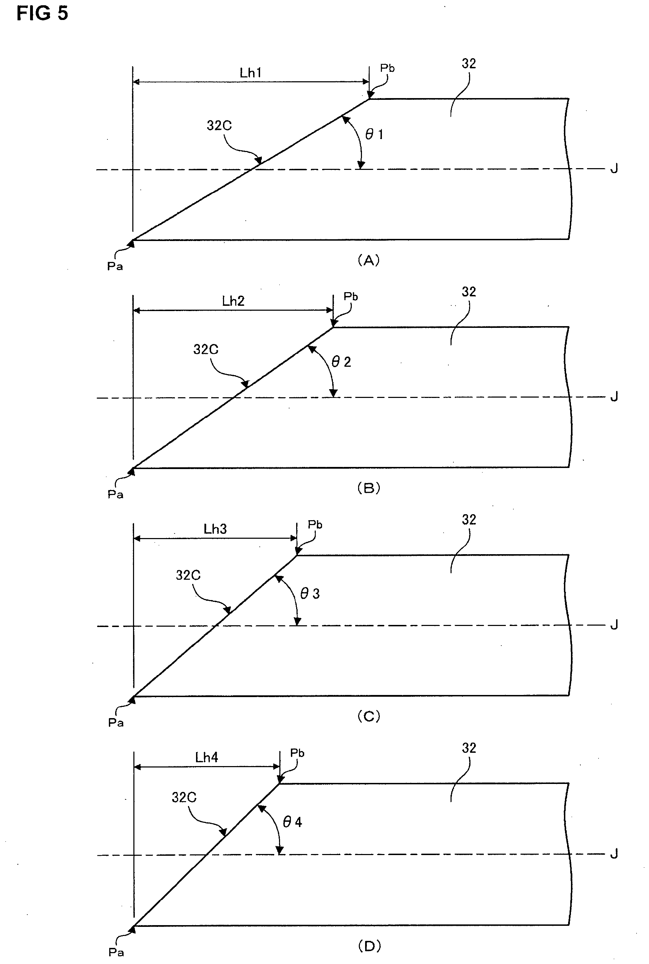

FIG. 5(A) to FIG. 5(D) are views showing specific examples when the tilt angle of the blade surface is changed. FIG. 5(A) shows a case when a tilt angle θ1 of the blade surface 32C is set to about 31°, and FIG. 5(B) shows a case when a tilt angle θ2 of the blade surface 32C is set to about 35°. Further, FIG. 5(C) shows a case when a tilt angle θ3 of the blade surface 32C is set to about 40°, and FIG. 5(D) shows a case when a tilt angle θ4 of the blade surface 32C is set to about 45°.

### (Length of the blade surface)

As is clarified from FIG. 5(A) to FIG. 5(D), when the tilt angle θ of the blade surface 32C becomes large, length Lh of the blade surface 32C is shortened accordingly. Specifically, length Lh2 of the blade surface 32C when the tilt angle of the blade surface 32C is θ2 ≈ 35° becomes shorter than the length Lh1 of the blade surface 32C at the time of θ1 ≈ 31°, and length Lh3 of the blade surface 32C when the tilt angle of the blade surface 32C is θ3 ≈ 40° becomes shorter than the length Lh2 of the blade surface 32C at the time of θ2 ≈ 35°. Also, length Lh3 of the blade surface 32C when the tilt angle of the blade surface 32C is θ3 ≈ 40° becomes shorter than the length Lh2 of the blade surface 32C at the time of θ1 ≈ 35°, and length Lh4 of the blade surface 32C when the tilt angle of the blade surface 32C is θ4 ≈ 45° becomes shorter than the length Lh3 of the blade surface 32C at the time of θ3 ≈ 40°. As shown in FIG. 4, the length (Lh) of the blade surface 32C described here, means the length of the blade surface 32C in a direction of a central axis J of the stabbing tube 32, and more specifically means the length of the blade surface 32C in this direction from one end Pa to the other end Pb.

The length Lh of the blade surface 32C may be set to a dimension so that a major part (preferably an entire part) of the discharging port 32D of the blade surface 32C fits into the blood vessel when the stabbing tube 32 stabs the blood vessel (retinal vein). Specifically, the length Lh of the blade surface 32C may be set in a range of 30 µm or more and 120 µm or less, in consideration of the fact that the thickness of the retinal vein is about a diameter of 100 µm.

### <2. Method of manufacturing the injection needle>

The method of manufacturing the injection needle 1 will be described next. First, the needle base 2, the main needle tube 31, the stabbing tube 32, and the reinforcement tube 33 are prepared as components of the injection needle 1. Among them, the component excluding the stabbing tube 32 can be fabricated by the same method (however, without forming the blade surface) as the method of fabricating the components of the injection needle for a general medical use. Therefore, the method of fabricating the stabbing tube 32 will be described here in detail.

When the stabbing tube 32 is fabricated, first, a metal tube having a circular cross-sectional face having a larger outer diameter than a target outer diameter d2, is fabricated. Specifically, for example, a thin plate of a stainless steel such as SUS304 is rounded and a seam portion is welded. At this time, the seam portion is polished as needed.

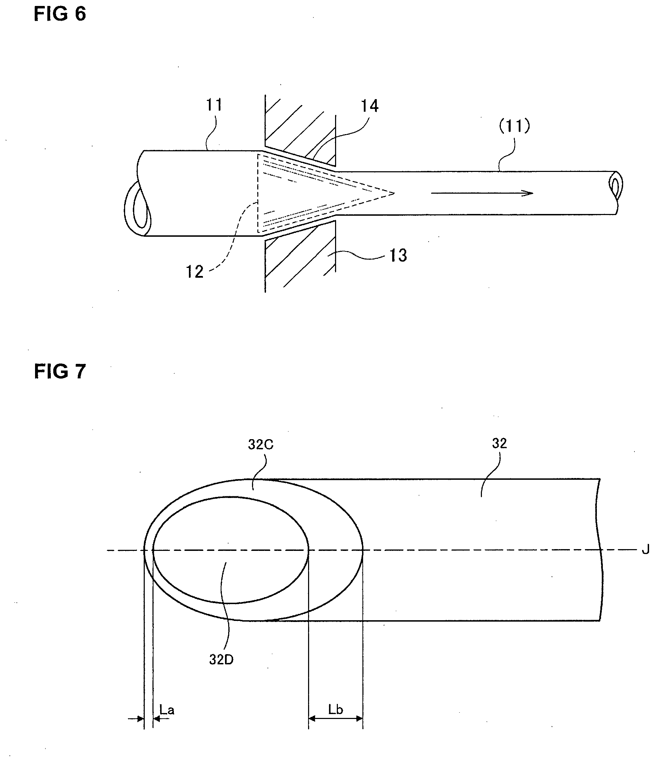

Next, the abovementioned metal tube is further thinned by a drawing process. Specifically, as shown in FIG. 6, a conical plug 12 is inserted into the metal tube 11, and the metal tube 11 is passed through a taper-shaped hole 14 provided on a die 13 along a conical surface of the plug 12. Then, the metal tube 11 is pulled-out from a large diameter side to a small diameter side of the hole 14. Thus, the outer diameter of the metal tube 11 pulled-out through the die 13 is more thinly narrowed to a dimension equivalent to an opening dimension of a small diameter side hole 14. Such a drawing process is repeated multiple numbers of times until the outer diameter of the metal tube 11 is thinned to about 0.5 mm. Thereafter, the outer diameter of the metal tube 11 is thinned to a desired dimension (for example, 50 µm) by repeating the drawing process multiple numbers of times in a state of not inserting the plug 12.

Next, the metal tube 11 is cut to a desired length. Then, the end portion of the metal tube 11 is diagonally cut by wire cut electric discharging or grinding, etc., thus obtaining the stabbing tube 32 with its cut surface formed as the blade surface 32C.

As a modified example of the method of manufacturing the injection needle 1, it is also acceptable to manufacture a needle integrally including the main needle tube 31 and the stabbing tube 32, by further applying the drawing process up to a prescribed place of the main needle tube 31.

An assembly procedure of the component of the injection needle 1 will be described next.

First, the main needle tube 31 is attached to the needle base 2. Specifically, the end portion of the main needle tube 31 is inserted into a through hole (not shown) formed on a tip side of the needle base 2, and thereafter a suitable amount of adhesive agent is supplied to the tip portion of the needle base 2 using a dispenser, etc. As the adhesive agent, for example, a thermosetting resin or a photocurable resin can be used. However, in this stage, the adhesive agent is set in an uncured state.

Next, the reinforcement tube 33 is fitted from the tip side of the main needle tube 31, with the end portion of the reinforcement tube 33 abut on the tip portion of the needle base 2 in contact with the adhesive agent. Thereafter, the adhesive agent is cured by heating or irradiation of a light (such as UV-ray, etc.), to thereby fix the main needle tube 31 and the reinforcement tube 33 together to the tip portion of the needle base 2.

Next, the end portion (portion supposed to be an insertion part 32B) of the stabbing tube 32 is inserted into the tip portion of the main needle tube 31. At this time, the end portion of the stabbing tube 32 is inserted into the main needle tube 31 so that the protruding part 32A of the stabbing tube 32 protrudes from the tip portion of the main needle tube 31 by about 0.5 mm for example. Thereafter, the stabbing tube 32 is fixed to the main needle tube 31 by laser welding or using the adhesive agent, etc. In a case of the laser welding, an outer circumferential surface of the main needle tube 31 surrounding the insertion part 32B of the stabbing tube 32 is irradiated with a laser beam, to thereby join an inner circumferential surface of the main needle tube 31 and the outer circumferential surface of the reinforcement tube 33 by melting. A plurality of sites (for example, three sites at an equal interval of 120°) in a circumferential direction of the main needle tube 31 may be set as irradiation sites of the laser beam.

### <3. Effect of this embodiment>

In the injection needle 1 according to an embodiment of the present invention, the following effect can be obtained.

### (First effect)

As the constitution of the needle tube 3, the tilt angle θ of the blade surface 32C of the stabbing tube 32 is set in a range of 30° < θ ≤ 45° which is larger than the injection needle for a general medical use. Thus, after satisfactorily securing a stabbing performance (easiness to stab) of the needle tube 3, the treatment liquid can be efficiently injected into the vein through the needle tube 3. Detailed explanation will be given hereafter.

First, the stabbing performance of the needle tube 3 will be described.

As described above, when the tilt angle θ of the blade surface 32C is set to be large, the sharpness of a point of the needle is lost. Therefore, in the same way as the injection needle for a general medical use, there is a concern that the stabbing resistance of the stabbing tube 32 becomes large, thus making it difficult to stab a target blood vessel. However, when the stabbing performance is confirmed using the injection needle 1 with the stabbing tube 32 actually fabricated by the inventors of the present invention, the stabbing tube 32 can stab the vein without any trouble. The reason can be considered as follows.

When the injection needle stabs the blood vessel using the injection needle for a general medical use, the injection needle stabs the blood vessel through a skin. Therefore, the stabbing resistance becomes large and the stabbing performance becomes poor when the needle point stabs the skin, unless the tilt angle of the blade surface of the injection needle is made small and the point of the needle is made sharp.

On the other hand, when the stabbing tube 32 stabs the retinal vein using the injection needle 1 of this embodiment, the stabbing tube 32 stabs the vein through a surface layer of the retina (inner limiting membrane) without passing through the skin. The surface layer of the retina is significantly thin compared to the skin, and the retinal vein is weak compared to the vein of an arm. Therefore, even if the tilt angle θ of the blade surface 32C is large to some extent, it is conceivable that a relatively easy stabbing can be performed.

An injection efficiency when injecting the treatment liquid into the retinal vein, will be described next.

In order to increase the injection efficiency of the treatment liquid, it is important to prevent the discharging port 32D of the stabbing tube 32 from protruding out of the vein when the stabbing tube 32 stabs the vein. In this point, when the tilt angle θ of the blade surface 32C is set to be large as described above, the length Lh of the blade surface 32C becomes shortened accordingly. Therefore, when the stabbing tube 32 stabs the vein, it becomes easy to fit the discharging port 32D of the blade surface 32C into the vein. Accordingly, even in a case of an ultrathin blood vessel like the retinal vein, the needle tube 3 can stab the blood vessel and the treatment liquid can be efficiently injected into the blood vessel through the needle tube 3. Particularly, when the length Lh of the blade surface 32C is set in a range of 30 µm ≤ Lh ≤ 120 µm, the discharging port 32D of the blade surface 32C can be fitted into the vein without allowing the needle point to interfere with a vessel wall at a depth side in a staging direction.

As described above, after satisfactorily securing the stabbing performance of the needle tube 3, the treatment liquid can be efficiently injected into the vein through the needle tube 3.

### (Second effect)

According to the injection needle 1 of this embodiment of the present invention, a damage added on the vein can be reduced for the following reason.

When the stabbing tube 32 stabs the vein to inject the treatment liquid, the treatment liquid is required to pass through the ultrathin stabbing tube 32. Therefore, the treatment liquid must be pushed out by adding a certain degree of strong pressure, using a pressurizing means not shown. Accordingly, the treatment liquid rushes out with great force from the discharging port 32D of the stabbing tube 32. In such a case, there is a problem that the vein receives a damage under power of the treatment liquid. More detailed explanation will be given hereafter.

First, when the surface layer side (inner limiting membrane side) of the retina is set as an upper side, and the opposite side thereof is set as a lower side, the discharging port 32D is faced upward when the stabbing tube 32 stabs the retinal vein. At this time, if the tilt angle θ of the blade surface 32C is set in a range of 15° to 20° similarly to a normal injection needle, the treatment liquid rushed out from the discharging port 32D of the blade surface 32C, is easily advanced toward the upper side blood vessel wall. Accordingly, the vein easily receives the damage under power of the treatment liquid.

On the other hand, when the tilt angle θ of the blade surface 32C is set in a range of 30° < θ ≤ 45°, the blade surface 32C is in a rise state inside of the vein by a portion that the tilt angle θ becomes large. Therefore, when an angle formed by the stabbing tube 32 (stabbing angle) at the time of stabbing the retinal vein becomes large, the treatment liquid rushed out from the discharging port 32D of the blade surface 32C is easily advanced along the blood vessel wall. Accordingly, the pressure becomes weak when the tilt angle of the blade surface 32C is set to be large, compared to the pressure that acts on the blood vessel wall when the treatment liquid is discharged. Therefore, the damage added on the vein becomes small.

### (Third effect)

According to the injection needle 1 of the embodiment of the present invention, it becomes possible to suitably respond to particularly the treatment of the central vein occlusion out of the retinal vein occlusion. More detailed description will be given hereafter.

The central vein occlusion is a disease in which the vein is clogged in literally the center of the retina (near the optic nerve head). The retinal vein is spread throughout the retina in an arcade shape from the center of the retina. Therefore, when clogging occurs in the center of the retina (a root portion of the blood vessel), its influence extends to an entire retina, thus increasing an adverse effect on vision. Accordingly, it is significantly effective to solve the clogged blood vessel by injecting the treatment liquid (such as thrombolytic agents, etc.). However, veins are extended in a plurality of directions from the center of the retina, in a meandering state. Therefore, a direction of the vein is different depending on its position, even in a case of the vein at the center of the retina.

On the other hand, for example as shown in the above prior art specification, when the needle tube 3 is set in a bent state in the middle, it is sometimes difficult to set the needle tip portion (stabbing tube 32) of the injection needle along the direction of the vein, depending on the direction of the clogged vein. Further, when the needle tube 3 is bent, it is difficult to pass the needle tube 3 through a cannula, and the needle tip portion can hardly stab the vein in some cases.

On the other hand, the injection needle 1 of this embodiment employs a constitution in which the needle tube 3 is formed straight. When such a constitution is employed, the angle (stabbing angle) formed by stabbing the vein of the center of the retina by the stabbing tube 32, becomes significantly large. Specifically, the stabbing angle is in a range of 45° or more and 90° or less, with a result that the stabbing tube 32 stabs the vein from an almost vertical direction. At this time, even if the tilt angle θ of the blade surface 32C of the stabbing tube 32 is large, the vein or a film covering the vein is significantly weak, and therefore the tip of the stabbing tube 32 can easily stabs the vein. Further, since the length Lh of the blade surface 32C is shortened, the discharging port 32D is easily fitted into the vein. Moreover, when the bent needle tube 3 is used, there is a necessity for setting the direction of the stabbing tube 32 along the direction of a target vein, but there is no necessity thereof when the needle tube 3 is formed straight like this embodiment. Further, since the needle tube 3 extends straight toward the needle point from the needle base, the needle point (stabbing tube 32) can accurately stab the target vein. Accordingly, this embodiment can suitably respond to the treatment of the central vein occlusion.

### <4. Other embodiment>

FIG. 7 is a view of an essential part of the injection needle according to the present invention. In FIG. 7, the position of the discharging port 32D in the blade surface 32C of the stabbing tube 32 is biased to the needle point side. Specifically, the needle point side length La of the blade surface 32C is shorter than the needle base side length Lb of the blade surface 32C in the direction of the central axis J of the stabbing tube 32. Therefore, the edge of the discharging port 32D is positioned nearest to the needle point of the stabbing tube 32. The ratio of each length La and Lb is preferably set in a range so that the length Lb is 1 or more and 4 or less when the length La is 1.0.

The injection needle having such a constitution can be obtained by diagonally cutting the end portion of the metal tube 11 and thereafter chemically polishing the cut surface.

When the treatment liquid is injected to the retinal vein using the injection needle having the abovementioned constitution, the discharging port 32D can be fitted into the vein even if not deeply stabbing the vein by the stabbing tube 32. Therefore, the needle point of the stabbing tube 32 hardly interferes with the blood vessel wall of the vein. Accordingly, the treatment of the retinal vein occlusion can be performed more safely.

Incidentally, it is a matter of course that the constitution in which the positon of the discharging port 32D is biased to the blade tip side in the central axis direction of the stabbing tube 32, can be applied to a case when the tilt angle θ of the blade surface 32C is defined in a range of 30° < θ ≤ 45°, and a case when the length Lh of the blade surface 32C is defined in a range of 30 µm ≤ Lh ≤ 120 µm, but can be widely applied to the other injection needle. Preferable aspects in this case will be described hereafter.

### (Supplementary description)

There is provided an injection needle, including
a needle tube having a discharging port at a needle point, for discharging a treatment liquid,
the needle tube including a stabbing tube having a blade surface with the discharging port formed thereon, and a main needle tube which is thicker than the above needle tube, with the needle tube provided on a tip portion of the main needle tube,
wherein a position of the discharging port is biased to a needle tip side in the blade surface of the stabbing tube.

### <5. Modified example, etc.>

A technical range of the present invention is not limited to the abovementioned embodiment, and includes various modifications or improvements in a range capable of deriving a specific effect obtained by features of the invention and a combination of them.

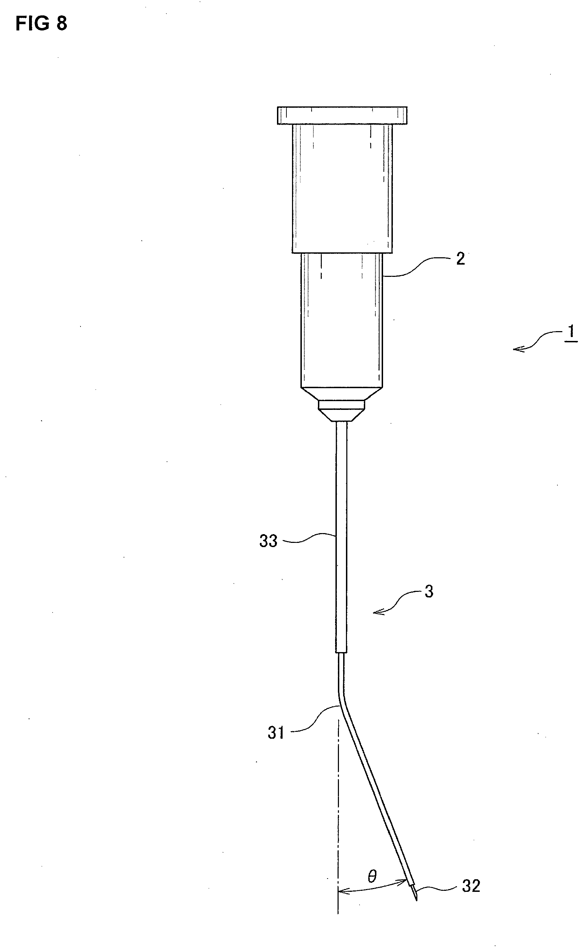

For example, the abovementioned embodiment shows a case in which the needle tube 3 is formed straight as an example, but the present invention is not limited thereto, and for example as shown in FIG. 8, the present invention is also applied to a case in which the needle tube 3 is bent in the middle or although not shown, a case in which the needle tube 3 is bent in an arch shape as a whole. In the treatment of the retinal vein occlusion, there is a case in which it is easy to use the injection needle with the needle tube 3 in a bending state, and a case in which it is easy to use the injection needle with the needle tube 3 formed straight, depending on the position or the direction of the target vein. Therefore, the injection needle having a different shape of the needle tube 3 may be selectively used, depending on the position or the direction of the target vein.

Further, in the abovementioned embodiment, the tilt angle θ of the blade surface 32C of the stabbing tube 32 is set in the range of 30° < θ ≤ 45°, and the length Lh of the blade surface 32C of the stabbing tube 32 is set in the range of 30 µm ≤ Lh ≤ 120 µm. However, these conditions are not required to be simultaneously satisfied, and only the first one may be satisfied.

Further, the abovementioned embodiment defines the tilt angle θ of the blade surface 32C of the stabbing tube 32 as "beyond 30° and 45° or less" as a particularly preferable embodiment example. However, it can be defined as "30° or more and 45° or less" as described in the following

### (Supplementary description 2)

There is provided an injection needle including a needle tube having a discharging port at a needle point for discharging a treatment liquid,
the needle tube including a stabbing tube having a blade surface with the discharging port formed thereon, and a main needle tube which is thicker than the stabbing tube, with the stabbing tube provided on a tip portion of the main needle tube,
wherein the stabbing tube satisfies a dimension condition such that an outer diameter is 70 µm or less (not including zero), and an inner diameter is 40 µm or less (not including zero), and a tilt angle of the blade surface with respect to a central axis of the stabbing tube satisfies a condition of 30° ≤ θ ≤ 45°.

Even in a case in which the stabbing tube is thus defined, the tilt angle θ of the blade surface 32C of the stabbing tube 32 is sufficiently large, compared to the tilt angle (15° to 20°) of the blade surface employed in the injection needle for a general medical use. Therefore, it is possible to realize the injection needle suitable for a use when injecting the treatment liquid into the retinal vein.

Further, in the abovementioned embodiment, when the injection needle 1 is manufactured, the metal tube having a desired outer diameter is cut to a desired length, and thereafter the needle point portion is subjected to wire cut electric discharging or grinding. However, the present invention is not limited thereto, and the following method can also be employed.

First, the metal tube thinned to a desired dimension (outer diameter) by the abovementioned drawing process, is cut to a length of two finally obtained stabbing tubes 32. Next, a longitudinal middle portion of the metal tube is diagonally cut by laser beam machining. Thus, two stabbing tubes 32 can be simultaneously obtained by cutting by a single laser beam radiation. At this time, as laser machining, non-thermal pulse laser machining, and more preferably femtosecond laser machining or picosecond laser machining is preferably employed. This is because when the pulse laser machining, etc., is employed, an edge portion of a cut section of the metal tube which is a base of the stabbing tube 32 can be formed sharp without sagging due to heat, thus making the stabbing resistance small. Further, in a case of polishing the tip of the stabbing tube 32 with a diameter thinned on the assumption that the blood vessel is thin having a thickness of about 100 µm, there is a problem of generation of polishing debris due to the thinness and clogging caused thereby. However, in a case of employing the pulse laser machining, etc., such a problem is not generated.

Further, in the laser machining described above, for example, by arranging a plurality of metal tubes having a length of two tubes in a supporting member not shown, and sequentially cutting these metal tubes by laser machining, a work can be efficiently advanced. Further, by supporting a cut-scheduled portion of the metal tube in a floating state using the abovementioned supporting member, and collecting the laser beams there to cut the metal tube, contamination of the cut section of the metal tube can be reduced. This is because when the cut-scheduled portion of the metal tube is set in the floating state and the laser beams are collected there, evaporation or scattering of a substance other than the metal tube is suppressed, and other substance is hardly adhered to the cut section of the metal tube.

Further, in a case of using the pulse laser machining, etc., the following manufacturing procedure can be employed. First, the metal tube obtained by the drawing process is cut into a length (for example 10 mm, etc.) that can be easy to be handled. Subsequently, a part of the cut metal tube is inserted into the main needle tube 31, and fixed by laser welding, etc. Thereafter, the tip portion of the metal tube is diagonally cut to a desired length by pulse laser machining, etc. By employing this procedure, the protruding part can be made small even in a case of the injection needle having the stabbing tube 32 with an outer diameter of 70 µm or less.

Further, in the abovementioned embodiment, the needle tube 3 attached to the needle base 2 has a three-stage constitution in which the main needle tube 31, the stabbing tube 32, and the reinforcement tube 33 are combined. However, the present invention is not limited thereto. Specifically, it is also acceptable that the needle tube 3 has a constitution formed only by the main needle tube of a single tube constitution without making a double tube constitution in which the main needle tube 31 and the reinforcement tube 33 are combined. In such a case, the following constitution may be employed: namely, a tapered (diagonal) stepped portion is formed in a longitudinally middle of the metal tube (main needle tube) by the abovementioned drawing process, and with this stepped portion as a border, a needle base side diameter of the tube is set to be large and a needle point side diameter of the tube is set to be small.

Further, it is also acceptable to employ the following constitution: namely, by incorporating a filter in the needle base 2 and capturing a fine foreign matter, etc., by this filter, the clogging in the needle tube 3 (particularly in the stabbing tube 32) is eliminated.

Further, although the abovementioned injection needle 1 is suitable for the use for stabbing the ultrathin blood vessel, the present invention is not limited thereto, and can be widely used for medical applications in general or other purposes.

### Description of Signs and Numerals

- 1: Injection needle
- 2: Needle base
- 3: Needle tube
- 31: Main needle tube
- 32: Stabbing tube
- 32A: Protruding part
- 32B: Insertion part
- 32C: Blade surface
- 32D: Discharging port
- 33: Reinforcement tube

## Claims

1. An injection needle (1), comprising a needle tube (3) having a discharging port (32D) at a needle point for discharging a treatment liquid,
the needle tube comprising a stabbing tube (32) having a blade surface (32C) with the discharging port (32D) formed thereon, and a main needle tube (31) which is thicker than the stabbing tube (32), with the stabbing tube (32) provided on a tip portion of the main needle tube, wherein the stabbing tube (32) satisfies a dimension condition that an outer diameter (d2) is 40 µm or more and 70 µm or less, and an inner diameter (d4) is 20 µm or more and 40 µm or less, and a tilt angle θ of the blade surface with respect to a central axis of the stabbing tube satisfies a condition of 30° < θ ≤ 45°, **characterised in that**
a position of the discharging port (32D) on the blade surface (32C) of the stabbing tube (32) is biased to a needle point side.

2. The injection needle according to claim 1, wherein a length Lh of the blade surface (32C) in a central axis direction of the stabbing tube (32) satisfies a condition of 30 µm ≤ Lh ≤ 120 µm.

3. The injection needle according to claim 1 or 2, wherein the needle tube (3) is formed straight.

## Patentansprüche

1. Eine Injektionsnadel (1), umfassend ein Nadelröhre (3) mit einer Ausstoßöffnung (32D) an einer Nadelspitze zum Ausstoßen einer Behandlungsflüssigkeit,
wobei die Nadelröhre eine Stechröhre (32) umfasst, die eine Klingenoberfläche (32C) aufweist mit darauf ausgebildeter Ausstoßöffnung (32D), und eine Hauptnadelröhre (31), die dicker ist als die Stechröhre (32), wobei die Stechröhre (32) an einem Spitzenabschnitt der Hauptnadelröhre bereitgestellt wird,
wobei
die Stechröhre (32) eine Bemaßungsbedingung erfüllt, die darin besteht, dass ein Außendurchmesser (d2) 40 µm oder mehr und 70 µm oder weniger beträgt und dass ein Innendurchmesser (d4) 20 µm oder mehr und 40 µm oder weniger beträgt, und dass ein Neigungswinkel θ der Klingenoberfläche in Bezug auf eine Mittelachse der Stechröhre folgende Bedingung erfüllt: 30° < θ ≤ 45°,
**dadurch gekennzeichnet, dass**
eine Position der Ausstoßöffnung (32D) auf der Klingenoberfläche (32C) der Stechröhre (32) zu einer Nadelspitzenseite versetzt (*biased*) ist.

2. Die Injektionsnadel nach Anspruch 1, wobei eine Länge Lh der Klingenoberfläche (32C) in einer Mittelachsenrichtung der Stechröhre (32) folgende Bedingung erfüllt: 30 µm ≤ Lh ≤ 120 µm.

3. Die Injektionsnadel nach Anspruch 1 oder 2, wobei die Nadelröhre (3) gerade ausgebildet ist.

## Revendications

1. Une aiguille d'injection (1), comprenant un tube d'aiguille (3) présentant un orifice de décharge (32D) au niveau d'une pointe d'aiguille pour la décharge d'un liquide de traitement,
le tube d'aiguille comprenant un tube de piquage (32) ayant une surface de lame (32C) avec l'orifice de décharge (32D) formé sur celle-ci, et un tube d'aiguille principal (31) qui est plus épais que le tube de piquage (32), le tube de piquage (32) étant prévu sur une portion d'extrémité du tube d'aiguille principal,
sachant que
le tube de piquage (32) satisfait à une condition de dimension selon laquelle un diamètre extérieur (d2) est de 40 µm ou plus et de 70 µm ou moins, et un diamètre intérieur (d4) est de 20 µm ou plus et de 40 µm ou moins, et un angle d'inclinaison θ de la surface de la lame par rapport à un axe central du tube de piquage satisfait à une condition de 30° < θ ≤ 45°,
**caractérisé en ce que**
une position de l'orifice de décharge (32D) sur la surface de lame (32C) du tube de piquage (32) est décalée vers un côté de pointe d'aiguille.

2. L'aiguille d'injection d'après la revendication 1, sachant qu'une longueur Lh de la surface de lame (32C) dans une direction d'axe central du tube de piquage (32) satisfait une condition de 30 µm ≤ Lh ≤ 120 µm.

3. L'aiguille d'injection d'après la revendication 1 ou 2, sachant que le tube d'aiguille (3) est formé droit.
